# EUROPEAN PATENT APPLICATION

(11) **EP 1 929 957 A1**
(43) Date of publication of application: **11.06.2008**
(21) Application number: 06796444.5
(22) Date of filing: 16.08.2006
(51) Int. Cl.: A61B 17/00

(54) **BALLOON TO BE PLACED IN STOMACH AND VEHICLE FOR BALLOON**

(30) Priority: 24.08.2005 JP 2005242040
(71) Applicant: OLYMPUS MEDICAL SYSTEMS CORP., Shibuya-ku Tokyo 151-0072 (JP)
(72) Inventor: KAJI, Kunihide, Tokyo, 1920023 (JP); YAMAMOTO, Tetsuya, Saitama, 3570044 (JP); MIKKAICHI, Takayasu, Tokyo, 1830033 (JP)
(74) Representative: von Hellfeld, Axel
(86) International application number: PCT/JP2006/316084
(87) International publication number: WO 2007/023717

(57) **Abstract**

An intragastric balloon (1) to be placed in an inflated state in the stomach (S) is provided including: a balloon body that is made of an extensible material capable of being inflated and contracted; and a balloon medium that is enclosed in the balloon body and that includes a non-compressible volume holding material inflating the balloon body to hold a predetermined volume, wherein the total specific gravity of the balloon body and the balloon medium is less than that of water (the stomach contents S1).

## Description

### TECHNICAL FIELD

The present invention relates to an intragastric balloon that is left in a stomach for a long period of time in order to cause weight loss in morbidly obese patients and a balloon medium that is enclosed in the balloon.

### BACKGROUND ART

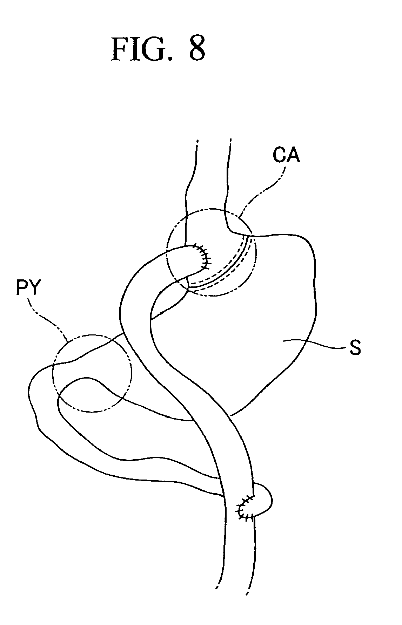

Conventionally, two methods are mainly considered for weight loss measures for morbidly obese patients, namely, a surgical treatment method and an endoscopic treatment method. One method is determined in accordance with a patient's preference and the treatment corresponding to the determined method is performed.
The surgical treatment method is to make a stomach smaller, for example, by surgery or to close the stomach S in the vicinity of the cardiac region CA and directly connect the digestive tract below the pyloric region PY to this point, as shown in FIG. 8. According to this method, the stomach capacity is restricted and it thus is possible to limit the amount of food that is ingested. In addition, a feeling of fullness can be obtained with only a small amount of food. Accordingly, it is possible to effectively obtain weight loss.
However, in this surgical treatment method, the burden on the patient is considerable, and because the stomach is excised and then rejoined, it is not possible to return to the original state, thereby not allowing the retrial to be performed again. Accordingly, this treatment is not one that can be undertaken easily by just anybody.

On the other hand, in the endoscopic treatment method, surgery is not required and thus the burden on the patient is small. Since this method has an advantage in that it is possible to perform the retrial again, this method currently attracts widespread attention.
In this endoscopic treatment method, by the use of an endoscope, a deflated balloon (i.e., a gastric balloon) is inserted into a stomach, is inflated inside the stomach, and is then left in an inflated state inside the stomach (see, for example, Patent Document 1 and Patent Document 2).

According to this method, since the balloon is left inside the stomach at all times, it is possible to limit the amount of food consumed in a single meal. As a result, weight loss can be effectively achieved.
In particular, since the endoscopic treatment method makes it possible to easily extract the balloon from the inside of the stomach by the use of an endoscope device, it is possible to repeatedly perform this method, as mentioned above. Accordingly, unlike in the surgical treatment method, this method has an advantage in that anybody can easily undergo the treatment.
Patent Document 1: U.S. Patent No. 4,694,827
Patent Document 2: U.S. Patent No. 5,084,061

### DISCLOSURE OF THE INVENTION

### Problems to be solved by the Invention

However, the conventional endoscopic treatment methods described in Patent Documents 1 and 2 have the following problems.
That is, conventionally, when a deflated balloon is inserted into a stomach and then inflated, the balloon is inflated by injecting pure water or physiological saline solution or the like into it. Accordingly, the inflated balloon becomes heavy and is left to press against the stomach wall of the stomach body to the pyloric region when the patient stands up. As a result, the stomach wall is damaged or the pressure is locally concentrated on a region contacting the stomach wall (which includes the pressure resulting from the gravity), thereby causing ulcers or bores in the stomach.

Since the balloon can be easily left to close the pyloric region, it becomes difficult for the contents of the stomach to pass to the digestive tract side, thereby causing a passage blockage. Since the heavy balloon is placed on the stomach wall, the patient tends to feel uncomfortable or nauseous.

In order to solve the above-mentioned problems, it has been considered to inflate the balloon by injecting gas into the balloon instead of the physiological saline solution. In this case, since the weight of the inflated balloon becomes smaller, it is possible to solve the above-mentioned problems. However, when a bore and the like is formed in the balloon in the stomach, the injected gas causes the balloon to explode, thereby excessively inflating the stomach. As a result, a great physical burden is imposed on the patient, which is also unsafe. Accordingly, it is not preferable to use gas to inflate the balloon.

The invention was conceived in view of the above-described problems of the conventional technology. It is an object of the invention to provide an intragastric balloon and a balloon medium that have no side effects such as ulcers, bore, or passage blockage of contents to effectively achieve weight loss and that can prevent an increase in pressure in the stomach even when a bore and the like is formed in the balloon.

### Means for Solving the Problems

In order to achieve the above-described object, the invention provides the following means.
According to a first aspect of the invention, there is provided an intragastric balloon that is left in an inflated state inside a stomach, the intragastric balloon including: a balloon body that is made of an extensible material capable of being inflated and contracted; and a balloon medium that is enclosed in the balloon body and that includes a non-compressible volume holding material inflating the balloon body to hold a predetermined volume, wherein the total specific gravity of the balloon body and the balloon medium is less than that of water.

In the intragastric balloon according to the aspect of the invention, the balloon body is inflated by the non-compressible volume holding material (for example, gel and the like) so as to have a predetermined volume, that is, a predetermined size and is maintained in this state. The balloon body is left in the inflated state inside a patient's stomach. Here, since the total specific gravity of the balloon body and the balloon medium is smaller than the specific gravity of water, the balloon floats over the contents conveyed by ingesting food. This is because the contents of the stomach include mixtures of various foods and much moisture such as gastric juice.

Accordingly, unlike the conventional heavy balloon, the balloon body does not come in contact with the stomach wall to press the stomach wall from the inside with a large pressure. As a result, since the stomach wall is not damaged and the pressure is not locally concentrated, it is possible to prevent the formation of ulcers or bores.
Since the balloon floats over the contents of the stomach, the balloon does not tend to close the pyloric region, unlike the conventional balloon. Accordingly, it is possible to prevent the passage blockage of the contents of the stomach. Unlike the conventional balloon, since the heavy balloon is not placed on the stomach wall, a patient hardly feels nauseous or uncomfortable.

Since the balloon is floating over the contents of the stomach, the balloon body can easily stimulate the fullness center of a stomach bottom with a small amount of food. As a result, a patient can achieve a feeling of fullness with a small amount of food. Since the balloon body is always left inside the stomach, the patient can limit the amount of food in one meal more easily. As a result, it is possible to satisfactorily treat the morbidly obese patient and to obtain excellent weight loss.

Particularly, the balloon body is inflated by the non-compressible volume holding material so as to hold a predetermined volume. Accordingly, even when a small bore is formed in the balloon body, the balloon is difficult to pop unlike the conventional balloon into which only compressed air is injected, and hardly inflates the stomach with an increase in pressure in the stomach. A material harmless to a human body can be used as the volume holding material.

As described above, since the intragastric balloon according to the aspect of the invention does not cause side effects such as ulcers, bores, and passage blockage of stomach contents, it is possible to obtain excellent weight loss. In addition, even when a bore is formed in the balloon, it is possible to prevent an increase in pressure in the stomach, thereby enhancing safety.

A second aspect of the invention provides the intragastric balloon according to the first aspect, wherein the volume holding material is a material that has an absorptive property and that is inflated by absorption.

In the intragastric balloon according to this aspect of the invention, since the volume holding material has an absorptive property and is inflated by absorption, such as natural cellulose, it is possible to satisfactorily inflate the balloon body. The volume holding material is inflated. Accordingly, even when a bore is formed in the balloon body, it is possible to hold a predetermined volume without deflating the balloon body.

A third aspect of the invention provides the intragastric balloon according to the first aspect, wherein the volume holding material is a solid formed in a predetermined shape and the balloon medium includes a plurality of the solids.

In the intragastric balloon according to this aspect of the invention, since the plurality of solids formed in a predetermined shape are enclosed in the balloon body, it is possible to satisfactorily inflate the balloon body. The solids are enclosed in the balloon body. Accordingly, even when a bore is formed in the balloon body, it is possible to hold the predetermined volume without deflating the balloon body. A material harmless to the human body can be used as the volume holding material.

A fourth aspect of the invention provides the intragastric balloon according to the third aspect, wherein the balloon medium includes a fluid that is enclosed in the balloon body along with the solids.

In the intragastric balloon according to this aspect of the invention, since a fluid such as physiological saline solution is enclosed in the balloon body so as to fill gaps between the plurality of solids, the surface of the balloon body can be made smooth, instead of uneven with respect to the solids. Accordingly, even when the balloon body comes in contact with the stomach wall, a patient hardly feels uncomfortable.

A fifth aspect of the invention provides the intragastric balloon according to the fourth aspect, wherein the fluid is a compressed gas that is capable of inflating the balloon body and of which the volume under the atmospheric pressure is smaller than the volume obtained by subtracting the volume of the plurality of solids from the volume of the stomach.

In the intragastric balloon according to this aspect of the invention, since the compressed gas is used as the fluid, it is possible to reduce the total specific gravity and thus to further reduce side effects such as ulcers, bores, and passage blockage of contents.
Particularly, the compressed gas is enclosed in the balloon body so that the volume under the atmospheric pressure is less than the volume obtained by subtracting the volume of the solids from the stomach volume. Accordingly, even when a bore is formed in the balloon body to pop the balloon body and the compressed gas is decompressed and discharged in the stomach, the stomach is not excessively inflated to a dangerous level. Accordingly, it is possible to guarantee safety.

According to a sixth aspect of the invention, there is provided an intragastric balloon that is left in an inflated state inside a stomach, the intragastric balloon including: a balloon body that is made of a non-extensible material; and a gas that is enclosed in a non-compressed state in the balloon body so that the balloon body has a predetermined volume, wherein the total specific gravity of the balloon body and the gas is smaller than the specific gravity of water.

In the intragastric balloon according to the aspect of the invention, the non-extensible balloon body is inflated by the gas enclosed in a non-compressed state so as to have a predetermined volume, that is, a predetermined size and is maintained in this state. The balloon body is left in the inflated state inside a patient's stomach. Here, since the total specific gravity of the balloon body and the balloon medium is less than the specific gravity of water, the balloon floats over the contents conveyed by ingesting food. This is because the contents of the stomach include mixtures of various foods and many fluids such as gastric juice.

Accordingly, unlike the conventional heavy balloon, the balloon body does not come in contact with the stomach wall to press the stomach wall from inside with a large amount of pressure. As a result, since the stomach wall is not damaged and the pressure is not locally concentrated, it is possible to prevent the formation of ulcers or bores.
Since the balloon floats over the contents of the stomach, the balloon does not tend to close the pyloric region, unlike the conventional balloon. Accordingly, it is possible to prevent the passage blockage of the contents of the stomach. Unlike the conventional balloon, since the heavy balloon is not placed on the stomach wall, a patient hardly feels nauseous or uncomfortable.

Since the balloon floats over the contents of the stomach, the balloon body can easily stimulate the fullness center of a stomach bottom with a small amount of food. As a result, a patient can achieve a feeling of fullness with a small amount of food. Since the balloon body is always left inside the stomach, the patient can limit the amount of food in one meal as much as possible. As a result, it is possible to satisfactorily treat morbidly obese patients and to obtain excellent weight loss.

Particularly, the balloon body is inflated by the non-compressed gas so as to hold a predetermined volume. Accordingly, even when a small bore is formed in the balloon body, it is difficult for the balloon to pop unlike the conventional balloon into which only the compressed air is injected, and hardly inflates the stomach with an increase in pressure in the stomach.
Since the balloon body is non-extensible, the balloon body can be easily deformed. Accordingly, when it comes in contact with the stomach wall, the balloon body is deformed and thus the pressure can be distributed over the stomach wall, thereby further reducing the risk of ulcers. Since the balloon body can be easily deformed in a state where it floats over the contents of the stomach, the balloon body does not affect the peristalsis of the stomach. Even when the ingested food is placed on the balloon body, the balloon body is deformed by means of the weight of the food to deliver the food to the pyloric region. Accordingly, it is possible to further prevent the passage blockage of contents.

As described above, since the intragastric balloon according to the aspect of the invention does not cause side effects such as ulcers, bores, and passage blockage of contents, it is possible to obtain an excellent weight loss effect. In addition, even when a bore is formed in the balloon, it is possible to prevent an increase in the pressure in the stomach, thereby enhancing safety.

According to a seventh aspect of the invention, there is provided a balloon medium being enclosed in an extensible balloon body that is left in an inflated state inside a stomach and including a non-compressible volume holding material that inflates the balloon body to a predetermined volume and holds the predetermined volume, wherein the total specific gravity of the balloon body and the balloon medium is less than the specific gravity of water.

In the balloon medium according to this aspect of the invention, the non-compressible volume holding material (for example, gel) inflates the balloon body so as to have a predetermined volume, that is, a predetermined size and holds this state. The balloon body is left in an inflated state inside a patient's stomach. Here, since the total specific gravity of the balloon body and the balloon medium is less than the specific gravity of water, the balloon floats over the contents conveyed by ingesting food. This is because the contents of the stomach include mixtures of various foods and many fluids such as gastric juice.

Accordingly, unlike the conventional heavy balloon, the balloon body does not come in contact with the stomach wall to press the stomach wall from inside with a large amount of pressure. As a result, since the stomach wall is not damaged and the pressure is not locally concentrated, it is possible to prevent the formation of ulcers or bores.
Since the balloon floats over the contents of the stomach, the balloon does not tend to close the pyloric region, unlike the conventional balloon. Accordingly, it is possible to prevent the passage blockage of the contents of the stomach. Unlike the conventional balloon, since the heavy balloon is not placed on the stomach wall, a patient hardly feels nauseous or uncomfortable, thereby preventing these feelings as much as possible.

Since the balloon floats over the contents of the stomach, the balloon body can easily stimulate the fullness center of a stomach bottom with a small amount of food.
As a result, a patient can achieve a feeling of fullness with a small amount of food. Since the balloon body is always left inside the stomach, the patient can limit the amount of food in one meal easily. As a result, it is possible to satisfactorily treat a morbidly obese patient and to obtain excellent weight loss.

Particularly, the balloon body is inflated by the non-compressible volume holding material so as to hold a predetermined volume. Accordingly, even when a small bore is formed in the balloon body, the balloon is difficult to pop unlike the conventional balloon into which only the compressed air is injected, and hardly inflates the stomach with an increase in pressure in the stomach. A material harmless to a human body can be used as the volume holding material.

As described above, since the balloon body inflated by the balloon medium according to the aspect of the invention does not cause side effects such as ulcers, bores, and passage blockage of contents, it is possible to obtain an excellent weight loss effect. In addition, even if a bore is formed in the balloon, it is possible to prevent an increase in pressure in the stomach, thereby enhancing safety.

According to an eighth aspect of the invention, there is provided a balloon medium being enclosed in a balloon body that is left in an inflated state inside a stomach and including a non-compressible volume holding material that inflates the balloon body to hold a predetermined volume, wherein the specific gravity is 1 or less.

In the balloon medium according to the aspect of the invention, the non-compressible volume holding material inflates the balloon body so as to have a predetermined volume, that is, a predetermined size and holds this state. Since the specific gravity of the balloon medium is less than or equal to 1 (the specific gravity of water), the inflated balloon body floats over the contents of the stomach. This is because the contents of the stomach include mixtures of various foods and many fluids such as gastric juice.

Accordingly, unlike the conventional heavy balloon, the balloon body does not come in contact with the stomach wall to press the stomach wall from inside with a large amount of pressure. As a result, since the stomach wall is not damaged and the pressure is not locally concentrated, it is possible to prevent the formation of ulcers or bores.
Since the balloon is floating over the contents of the stomach, the balloon does not tend to close the pyloric region, unlike the conventional balloon. Accordingly, it is possible to prevent the passage blockage of the contents of the stomach. Unlike the conventional balloon, since the heavy balloon is not placed on the stomach wall, a patient hardly feels nauseous or uncomfortable, thereby preventing such feelings as much as possible.

Since the balloon floats over the contents of the stomach, the balloon body can easily stimulate the fullness center of a stomach bottom with a small amount of food. As a result, a patient can achieve a feeling of fullness with a small amount of food. Since the balloon body is always left inside the stomach, the patient can limit the amount of food in one meal easily. As a result, it is possible to satisfactorily treat a morbidly obese patient and to obtain excellent weight loss.

Particularly, the balloon body is inflated by the non-compressible volume holding material so as to hold a predetermined volume. Accordingly, even when a small bore is formed in the balloon body, unlike the conventional balloon into which only the compressed air is injected, the pressure of the stomach does not increase suddenly due to the explosion to inflate the stomach. A material harmless to the human body can be used as the volume holding material.

As described above, since the balloon body inflated by the balloon medium according to this aspect of the invention does not cause side effects such as ulcers, bores, and passage blockage of contents, it is possible to obtain excellent weight loss. In addition, even when a bore is formed in the balloon, it is possible to prevent an increase in pressure in the stomach, thereby enhancing safety.

According to a ninth aspect of the invention, there is provided a balloon medium being enclosed in a balloon body that is left in an inflated state inside a stomach and including a non-compressible volume holding material that inflates the balloon body to hold a predetermined volume, wherein the balloon medium has a finite form.

In the balloon medium according to this aspect of the invention, since the volume holding material has a finite shape, it is possible to satisfactorily inflate the balloon body. Even when a bore is formed in the balloon body, it is possible to satisfactorily hold a predetermined volume without deflating the balloon body.

According to a tenth aspect of the invention, there is provided an intragastric balloon including the balloon medium according to the eighth or ninth aspect and a balloon body that be inflated by the balloon medium.

Since the intragastric balloon according to the aspect of the invention is inflated by the balloon medium, the balloon does not cause side effects such as ulcers, bores, and passage blockage of contents, it is possible to obtain excellent weight loss.
In addition, even when a bore is formed in the balloon, it is possible to prevent an increase in pressure in the stomach, thereby enhancing safety.

### Effects of the Invention

The intragastric balloon according to the invention does not cause side effects such as ulcers, bores, and passage blockage of contents, it is possible to obtain an excellent weight loss effect. In addition, even when a bore is formed in the balloon, it is possible to prevent an increase in pressure in the stomach, thereby enhancing safety.

### BRIEF DESCRIPTION OF THE DRAWINGS

FIG 1 is a diagram illustrating an intragastric balloon and a balloon medium according to a first embodiment of the invention, where the intragastric balloon is left in an inflated state inside a stomach.
FIG 2 is a diagram illustrating the intragastric balloon shown in FIG 1, where a deflated state of the intragastric balloon and an inflated state of the intragastric balloon which is inflated by a balloon medium are shown.
FIG 3 is a sectional view illustrating an insertion hole and a valve disposed in a balloon body of the intragastric balloon shown in FIG 1.
FIG. 4 is a diagram illustrating a state where the intragastric balloon shown in FIG 1 floats over contents of the stomach.
FIG 5 is a diagram illustrating an intragastric balloon and a balloon medium according to a second embodiment of the invention, where a state in which the intragastric balloon is inflated by the balloon medium including plural solids.
FIG 6A is a diagram illustrating solids having a shape different from cylindrical solids shown in FIG. 5, where granular solids are shown.
FIG. 6B is a diagram illustrating solids having a shape different from the cylindrical solids shown in FIG. 5, where granular solids having a space therein are shown.
FIG 7 is a sectional view illustrating an example of a valve different from the valve shown in FIG 3.
FIG 8 is a diagram illustrating a conventional surgical treatment method for a treatment for the morbidly obese, where the state of a stomach after operation is shown.

### Description of Reference Numerals and Signs

- S:: stomach
- 1, 10:: intragastric balloon
- 2:: balloon body
- 3:: natural cellulose (volume holding material)
- 4:: balloon medium
- 11:: solid (volume holding material)

### BEST MODE FOR CARRYING OUT THE INVENTION

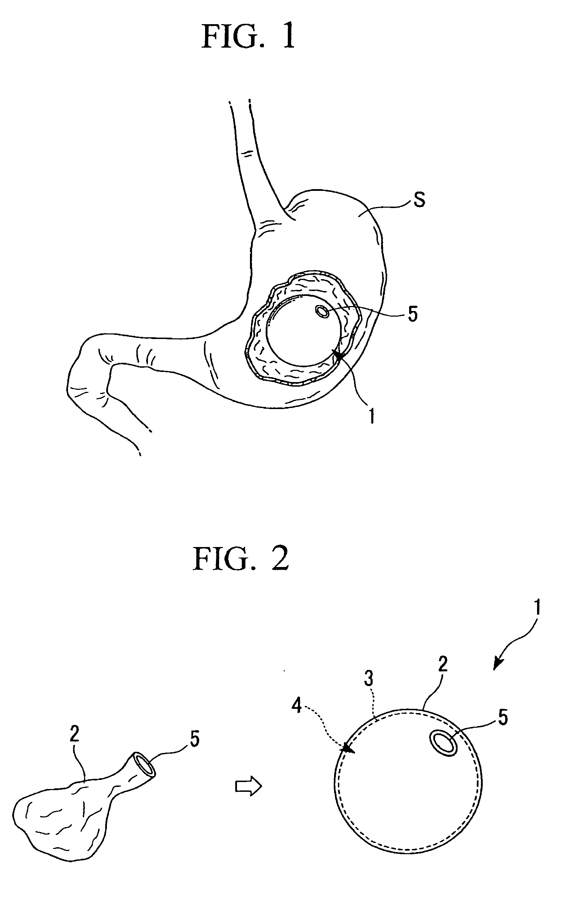

Hereinafter, an intragastric balloon and a balloon medium according to a first embodiment of the invention will be described with reference to FIGS. 1 to 4.
The intragastric balloon 1 according to this embodiment is left in an inflated state (the right side of FIG. 2) inside a stomach S as shown in FIGS. 1 and 2, and includes a balloon body 2 formed of an extensible material that can be inflated and deflated and a balloon medium 4 including natural cellulose (non-compressible volume holding material) 3 that is enclosed in the balloon body 2 and that inflates the balloon body 2 so as to hold a predetermined volume.

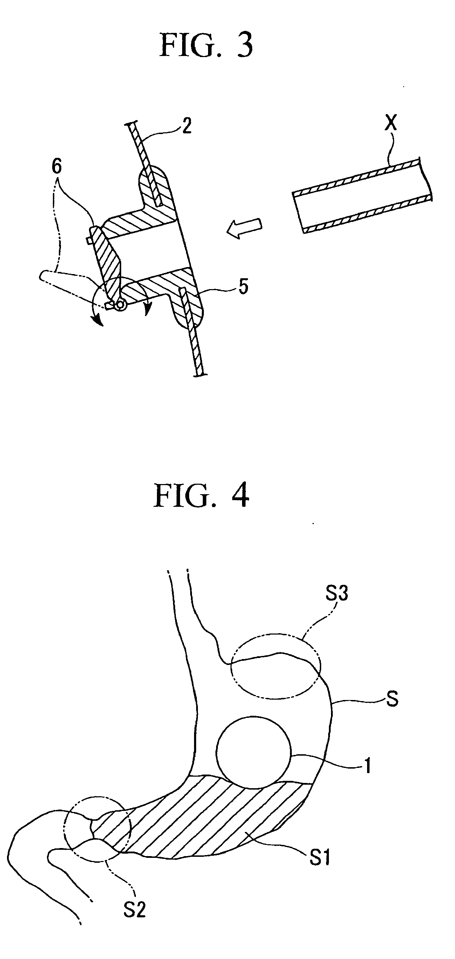

The balloon body 2 is inserted in a deflated state into a stomach S by an endoscope device not shown and is inflated in the stomach S by supplying the natural cellulose 3 thereto through a specific insertion tube X inserted into a channel of the endoscope device.
As shown in FIG. 3, the outer circumferential surface of the balloon body 2 is provided with an insertion hole 5 into which the insertion tube X is inserted. The insertion hole 5 is provided with a flap type valve 6 for automatically closing the opening of the insertion hole 5. The valve 6 is rotatably connected to the insertion hole 5 through a pin and serves to always close the opening by use of a spring not shown.
That is, when the insertion tube X is inserted into the insertion hole 5, the valve 6 is pushed by the tip of the insertion tube X and rotates to release the closed state of the opening of the insertion hole 5. When the insertion tube X is pulled out of the insertion hole 5, the valve 6 is urged by the spring to automatically close the opening of the insertion hole 5.

The natural cellulose 3 has an absorptive property and is a material that is inflated by absorption. That is, the natural cellulose 3 and water not shown and serving to inflate the natural cellulose 3 constitute the balloon medium 4. The total specific gravity of the balloon medium 4 and the balloon body 2 is adjusted to be less than that of water.

It will now be described how a morbidly obese patient is treated by the use of the intragastric balloon 1 having the above-mentioned configuration. In this embodiment, it is assumed that the compressed natural cellulose 3 is supplied to the balloon body 2 through the insertion tube X.
First, as shown in FIG 2, the deflated balloon body 2 is inserted into the stomach S by the use of the endoscope device not shown. The insertion tube X is inserted into a channel of the endoscope device after inserting the balloon body 2 and the insertion tube X is inserted into the insertion hole 5 of the balloon body while watching an endoscopic image, as shown in FIG. 3. At this time, since the valve 6 is opened by insertion of the insertion tube X, the inside of the balloon body 2 communicates with the insertion tube X.

After the insertion of the insertion tube X, the compressed natural cellulose 3 is supplied to the balloon body 2 through the insertion tube X and a predetermined amount of water is supplied thereto after the supply of the natural cellulose 3. Accordingly, the natural cellulose 3 starts absorbing the water and is slowly inflated in the balloon body 2. As a result, as shown in FIG 2, the balloon body 2 is slowly inflated up to a predetermined volume, that is, a predetermined size, with the inflation of the natural cellulose 3. Due to the cellulose 3 being inflated, the state of the balloon body 2 being inflated up to a predetermined volume is maintained. The natural cellulose 3 is inflated in a state where moisture and air are mixed by absorption.

The insertion tube X is pulled out of the insertion hole 5 after the inflation of the balloon body 2 and the endoscope device including the insertion tube X is removed from the patient's body. Accordingly, the intragastric balloon 1 is left in the inflated state in the patient's stomach S, as shown in FIG 1. By pulling out the insertion tube X, the valve 6 of the balloon body 2 is urged by a spring to automatically close the opening of the insertion hole 5. Accordingly, the inside of the balloon body 2 does not communicate with the outside through the insertion hole 5.

In the intragastric balloon 1 inside the stomach S, since the total specific gravity of the balloon body 2 and the balloon medium 4 is less than the specific gravity of water, the intragastric balloon floats over the various contents S1 conveyed to the stomach S by ingesting food, as shown in FIG 4. This is because the contents S1 of the stomach S include mixtures of various foods and much moisture such as gastric juice.
Accordingly, unlike the conventional heavy balloon, the balloon body 2 does not come in contact with the stomach wall to press the stomach wall from the inside with a large pressure. As a result, since the stomach wall is not damaged and the pressure is not locally concentrated, it is possible to prevent the formation of ulcers or bores.

Since the intragastric balloon 1 floats over the contents S1 of the stomach S, the intragastric balloon does not tend to close the pyloric region S2, unlike the conventional intragastric balloon. Accordingly, it is possible to prevent the passage blockage of the contents S1. Unlike the conventional balloon, since the heavy balloon is not placed on the stomach wall, a patient hardly feels nauseous or uncomfortable.
Since the intragastric balloon floats over the contents S1 of the stomach S, the balloon body 2 can easily stimulate the fullness center located on a stomach bottom S3 with a small amount of food. As a result, a patient can achieve a feeling of fullness with a small amount of food. Since the balloon body 2 is always inside the stomach S, the patient can limit the amount of food in one meal easily. As a result, it is possible to satisfactorily treat a morbidly obese patient and to obtain excellent weight loss.

The non-compressible natural cellulose 3 is inflated in the balloon body 2. Accordingly, even when a bore is formed in the balloon body 2, it is possible to hold a predetermined volume without deflating the balloon body 2. Unlike the conventional intragastric balloon into which only the compressed air is injected, the intragastric balloon is difficult to pop and hardly increases the pressure in the stomach to excessively inflate the stomach.

As described above, since the intragastric balloon 1 according to this embodiment inflated by the balloon medium 4 does not cause side effects such as ulcers, bores, and passage blockage of contents S1, it is possible to obtain excellent weight loss. In addition, even when a bore is formed in the balloon body 2, it is possible to prevent an increase in pressure in the stomach, thereby enhancing safety.
Although it has been described in the above-mentioned embodiment that the compressed natural cellulose 3 is supplied into the balloon body 2 by the insertion tube X, the compressed natural cellulose 3 may be disposed in advance into the balloon body 2.
The natural cellulose 3 (sponge type) disposed in advance in the balloon body 2 may be compressed to sufficient size to be orally inserted by making the inside of the balloon body 2 a negative pressure and may be decompressed into a balloon shape (inflated state) by means of the restoring force of the natural cellulose 3 by releasing the negative pressure and subjecting it to atmospheric pressure. In this case, the same operational advantages can be obtained.

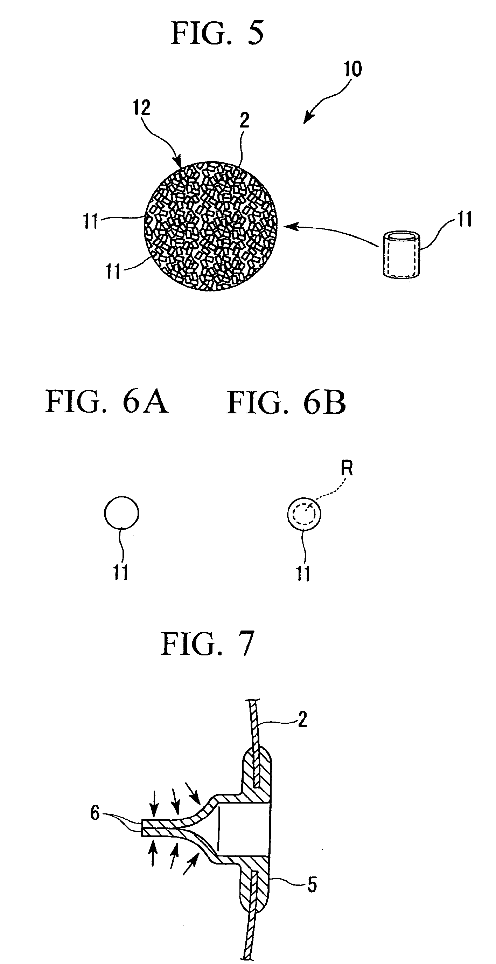

Next, an intragastric balloon and a balloon medium according to a second embodiment of the invention will be described with reference to FIG 5. In the second embodiment, the same elements as the first embodiment are denoted by the same reference numerals and the description thereof is omitted.
The second embodiment is different from the first embodiment, in that the intragastric balloon 1 according to the first embodiment employs the natural cellulose 3 as the non-compressible volume holding material for inflating the balloon body 2, but the intragastric balloon 10 according to the second embodiment employs solids 11 as the volume holding material.

That is, the volume holding material according to this embodiment, as shown in FIG 5, is the solid 11 formed in a cylindrical shape (predetermined shape). The solids 11 are harmless to the human body and are made of bio-absorptive materials and edible powders such as wheat bran and wheat flour. The balloon medium 12 according to this embodiment includes plural solids 11.

In the intragastric balloon 10 having the above-mentioned configuration, similarly to the first embodiment, since the plural solids 11 are enclosed in the balloon body 2, it is possible to satisfactorily inflate the balloon body 2 so as to hold a predetermined volume. The balloon body 2 can be inflated by supplying the plural solids 11 into the compressed balloon body 2 through the insertion tube X.
Alternatively, a method of injecting gas into the balloon body 2 through the insertion tube X with a pressure greater than or equal to the atmospheric pressure, supplying the solids 11 into the inflated balloon body 2, and opening the pressure of the balloon body 2 (setting the pressure to be equal to or less than the atmospheric pressure) after supplying enough solids 11 to hold the shape of the balloon body 2, may be employed.
Similarly to the first embodiment, even when a bore is formed in the balloon body 2, it is possible to hold the predetermined volume without deflating the balloon body 2 due to the plural solids 11 enclosed therein. The other operational advantages are equal to those of the first embodiment.

Although it has been described in the second embodiment that the solids 11 have a cylindrical shape, the invention is not limited to the shape. They may have a granular shape (pellet shape) as shown in FIG. 6A or may have a granular shape having a space R therein like a ping-pong ball as shown in FIG 6B. In this way, (the solids 11 are not limited in shape, but have a constant finite shaping property), by forming a space in the solids 11, it is possible to hold the shape with a small mass. Preferably, when the specific gravity of the solids 11 is 1 or more, it is easy to restrict the total specific gravity of the balloon body 2 to 1 or more.

In the second embodiment, an auxiliary fluid such as physiological saline solution or compressed gas may be supplied to and enclosed in the balloon body 2 along with the plural solids 11. That is, the plural solids 11 and the fluid may constitute the balloon medium 4.
Accordingly, since the fluid is enclosed in the balloon body 2 so as to fill the gaps between the plural solids 11, the surface of the balloon body 2 can be made to be smooth, not to have an uneven shape corresponding to the shapes of the solids 11. Accordingly, even when the intragastric balloon comes in contact with the stomach wall, the patient hardly feels uncomfortable.

Particularly, when compressed gas is used as the fluid, the volume of the compressed gas under the atmospheric pressure is less than the volume obtained by subtracting the volume of the plural solids 11 from the stomach volume. Accordingly, even when a bore is formed in the balloon body 2 to explode the balloon body and the compressed gas is inflated and discharged in the stomach, the stomach S is not excessively inflated into a dangerous state. Therefore, safety can be guaranteed. By using the compressed gas as the fluid, it is possible to reduce the total specific gravity, thereby preventing side effects such as an ulcer, a bore, or the passage blockage of contents S1.

When the compressed gas is used along with the plural solids 11, the pressure of the balloon body 2 slightly increases. Accordingly, the valve 6 fitted to the insertion hole 5 of the balloon body 2 may employ a duckbill type valve 6 that is automatically closed by an inner pressure as shown in FIG 7, instead of the flap type valve shown in FIG. 3.

The invention is not limited to the above-mentioned embodiments, but may be modified in various forms without departing from the gist of the invention.

For example, although the extensible balloon body has been used in the above-mentioned embodiments, the invention is not limited to such a case. The intragastric balloon may include a balloon body formed of a non-extensible material. That is, a balloon body like a simple vinyl bag may be employed. In this case, gas can be enclosed in a non-compressed state in the balloon body so that the balloon body has a predetermined volume. At this time, the total specific gravity of the balloon body and the gas is controlled to be smaller than the specific gravity of water.

According to the intragastric balloon having the above-mentioned configuration, it is possible to obtain the same operational advantages as the first embodiment.
Particularly, since the balloon body is inflated by the non-compressed gas so as to hold a predetermined volume, unlike the conventional case in which only the compressed gas is inserted, the balloon body is not exploded and does not increase the pressure of the stomach to excessively inflate the stomach, even when a small bore is formed in the balloon body.

The balloon body is non-extensible and thus can be easily deformed. Accordingly, when the balloon body comes in contact with the stomach wall, the balloon body can be deformed to distribute the pressure on the stomach wall, thereby reducing the risk of an ulcer. Since the balloon body can be easily deformed even in a state where it is floating over the contents of the stomach, it does not affect the peristalsis of the stomach. In addition, even when the ingested food is placed on the balloon body, the balloon body is deformed with the weight of the food. Therefore, it is possible to further prevent the passage blockage of the contents.

## Claims

1. An intragastric balloon that is left in an inflated state inside a stomach, the intragastric balloon comprising:
a balloon body that is made of an extensible material capable of being inflated and contracted; and
a balloon medium that is enclosed in the balloon body and that includes a non-compressible volume holding material inflating the balloon body to hold a predetermined volume,
wherein the total specific gravity of the balloon body and the balloon medium is less than that of water.

2. The intragastric balloon according to claim 1, wherein the volume holding material is a material that has an absorptive property and that is inflated by absorption.

3. The intragastric balloon according to claim 1, wherein the volume holding material is a solid formed in a predetermined shape, and
wherein the balloon medium includes a plurality of the solids.

4. The intragastric balloon according to claim 3, wherein the balloon medium includes a fluid that is enclosed in the balloon body along with the solids.

5. The intragastric balloon according to claim 4, wherein the fluid is a compressed gas that is capable of inflating the balloon body and of which the volume under the atmospheric pressure is less than the volume obtained by subtracting the volume of the plurality of solids from the volume of the stomach.

6. An intragastric balloon that is left in an inflated state inside a stomach, the intragastric balloon comprising:
a balloon body that is made of a non-extensible material; and
a gas that is enclosed in a non-compressed state in the balloon body so that the balloon body has a predetermined volume,
wherein the total specific gravity of the balloon body and the gas is less than the specific gravity of water.

7. A balloon medium being enclosed in an extensible balloon body that is left in an inflated state inside a stomach and including a non-compressible volume holding material that inflates the balloon body to a predetermined volume and holds the predetermined volume,
wherein the total specific gravity of the balloon body and the balloon medium is less than the specific gravity of water.

8. A balloon medium being enclosed in a balloon body that is left in an inflated state inside a stomach and including a non-compressible volume holding material that inflates the balloon body to hold a predetermined volume,
wherein the specific gravity is 1 or less.

9. A balloon medium being enclosed in a balloon body that is left in an inflated state inside a stomach and including a non-compressible volume holding material that inflates the balloon body to hold a predetermined volume,
wherein the balloon medium has a finite form.

10. An intragastric balloon comprising:
the balloon medium according to claim 8 or 9; and
a balloon body that be inflated by the balloon medium.
